# EUROPEAN PATENT SPECIFICATION

(11) **EP 2 938 625 B1**
(45) Date of publication and mention of the grant of the patent: **21.02.2018**
(21) Application number: 13869498.9
(22) Date of filing: 30.12.2013
(51) Int. Cl.: C07J 43/00

(54) **PROCESS FOR THE PREPARATION OF ABIRATERONE ACETATE**
VERFAHREN ZUR HERSTELLUNG VON ABIRATERONACETAT
PROCÉDÉ DE PRÉPARATION D'ACÉTATE D'ABIRATÉRONE

(30) Priority: 31.12.2012 IN 5538CH2012
(43) Date of publication of application: 04.11.2015
(73) Proprietor: Hetero Research Foundation, Hyderabad 500018, Andhra Pradesh (IN)
(72) Inventor: PARTHASARADHI REDDY, Bandi, Hyderabad 500018 Andhra pradesh (IN); RATHNAKAR REDDY, Kura, Hyderabad 500018 Andhra pradesh (IN); MURALIDHARA REDDY, Dasari, Hyderabad 500018 Andhra pradesh (IN); SRINIVASA REDDY, Katham, Hyderabad 500018 Andhra pradesh (IN); VAMSI KRISHNA, Bandi, Hyderabad 500018 Andhra pradesh (IN)
(74) Representative: Best, Michael
(86) International application number: PCT/IN2013/000817
(87) International publication number: WO 2014/102833

(56) References cited:
- WO-A1-2006/021777
- US-A- 5 604 213
- US-A1- 2011 312 916
- LEO A. PAQUETTE ET AL: "Enantioselective total synthesis of (-)-subergorgic acid", JOURNAL OF THE AMERICAN CHEMICAL SOCIETY, vol. 115, no. 1, 1 January 1993 (1993-01-01), pages 49-56, XP55284712, US ISSN: 0002-7863, DOI: 10.1021/ja00054a007
- JEFFREY D. WINKLER ET AL: "The First Total Synthesis of ( )-Saudin", JOURNAL OF THE AMERICAN CHEMICAL SOCIETY, vol. 121, no. 32, 1 August 1999 (1999-08-01), pages 7425-7426, XP55284708, US ISSN: 0002-7863, DOI: 10.1021/ja9916198

## Description

### Filed of the Invention

The present invention provides a novel process for the preparation of abiraterone. The present invention also provides a novel process for the preparation of abiraterone acetate.

### Background of the Invention

Abiraterone acetate is.chemically, 3β-acetoxy-17-(3-pyridyl)androsta-5,16-diene and has the structural formula:

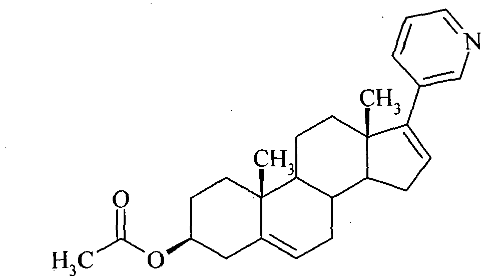

Abiraterone acetate is a drug used in castration-resistant prostate cancer (formerly hormone-resistant or hormone-refractory prostate cancer), prostate cancer not responding to androgen deprivation or treatment with antiandrogens. Abiraterone acetate is currently marketed under the trade name ZYTIGA® by Janssen Biotech.

Abiraterone acetate and its process were disclosed in U.S. patent no. 5,604,213 ('213 patent). According to the '213 patent, abiraterone acetate can be prepared by reacting diethyl(3-pyridyl)borane with 3β-acetoxyandrosta-5,16-dien-17-yl trifluoromethane sulphonate in tetrahydrofuran containing bis(triphenylphosphine)palladium(II) chloride and added aqueous solution of sodium carbonate and then concentrated. And the abiraterone acetate was separated by chromatography on elution with light petroleum-diethyl ether (2:1).

According to the '213 patent, 3β-acetoxyandrosta-5,16-dien-17-yl trifluoromethane sulphonate can be prepared by reacting dehydroepiandrosterone-3-acetate in dry dichlormethane containing 2,6-di-t-butyl-4-methylpyridine with trifluoromethanesulphonic anhydride and then separated by chromatography on elution with light petroleum-dichloromethane (3:1).

International application publication no. WO 1995/09178 described a process for the preparation of abiraterone acetate. According to the patent, abiraterone acetate can be prepared by reacting abiraterone in dry diethyl ether containing triethylamine and dimethylaminopyiridine with acetyl chloride and then recrystallized with ethanol and water.

U.S. patent no. 8,236,946 ('946 patent) disclosed a process for the preparation of 3β-acetoxyandrosta-5,16-dien-17-yl trifluoromethane sulphonate. According to the '946 patent, 3β-acetoxyandrosta-5,16-dien-17-yl trifluoromethane sulphonate can be prepared by reacting dehydroepiandrosterone-3-acetate in dichloromethane containing a base is selected from the group consisting of pyridine, 2,6-lutidine, N-methylmorpholine, 1,4-diazabicyclo[2.2.2]octane, triethylamine, triethylamine, N,N-diisopropylethylamine, quinclidine and 1,8-diazabicyclo[5.4.0]undec-7ene with trifluoromethanesulphonic anhydride and then concentrated.

It has been found that the preparation of abiraterone acetate by column isolation in the prior art. It is not commercially possible. The present invention makes now available a more efficient process for the preparation of abiraterone acetate.

We have found that the use of various bases such as 2,6-di-t-butyl-4-methylpyridine, pyridine, 2,6-lutidine, N-methylmorpholine, 1,4-diazabicyclo[2.2.2]octane, triethylamine, triethylamine, N,N-diisopropylethylamine, quinclidine and 1,8-diazabicyclo[5.4.0]undec-7ene for the preparation of abiraterone results in low yields. According to the present invention abiraterone can be obtained in higher yields than the prior art processes.

Thus, one object of the present invention is to provide a novel process for the preparation of abiraterone.

Another object of the present invention is to provide a novel process for the preparation of abiraterone acetate.

### Summary of the Invention

In one aspect, the present invention provides a novel process for the preparation of abiraterone, which comprises:
a) reacting dehydroepiandrosterone-3-acetate in a chlorinated solvent with trifluoromethanesulfonic anhydride in the presence of a base selected from the group consisting of n-methylpyrrolidone, N-methylpiperidine or tetramethylethylenediamine in a chlorinated solvent;
b) concentrating the reaction mass to obtain a residual mass;
c) reacting the residual mass obtained in step (b) in an ether solvent with diethyl(3-pyridyl)borane in the presence of bis(triphenylphosphine)palladium(II) chloride;
d) adding a base and water to the reaction mass;
e) concentrating the reaction mass to obtain a residual mass;
f) adding a base and an alcoholic solvent to the residual mass obtained in step (e);
g) adding water to the reaction mass;
h) pH of the reaction mass was adjusted with hydrochloric acid;
i) adding chlorinated solvent to the reaction mass;
j) removing the solvent from the reaction mass to obtain a residual solid;
k) slurring the residual solid obtained in step (j) with a hydrocarbon solvent; and
l) isolating the abiraterone.

In another aspect, the present invention provides a novel process for the preparation of abiraterone acetate, which comprises:
a) reacting abiraterone in a chlorinated solvent with acetic anhydride in the presence of pyridine and 4-dimethylaminopyridine;
b) adding sodium bicarbonate solution to the reaction mass;
c) concentrating the reaction mass to obtain a residual solid;
d) dissolving the residual solid in a nitrile solvent, an alcoholic solvent or mixture thereof;
e) heating the solution; and
f) isolating the abiraterone acetate.

### Detailed Description of the Invention

The term "room temperature" refers to temperature at about 25 to 35°C.

According to one aspect of the present invention, there is provided a novel process for the preparation of abiraterone, which comprises:
a) reacting dehydroepiandrosterone-3-acetate in a chlorinated solvent with trifluoromethanesulfonic anhydride in the presence of a base selected from the group consisting of n-methylpyrrolidone, N-methylpiperidine or tetramethylethylenediamine in a chlorinated solvent;
b) concentrating the reaction mass to obtain a residual mass;
c) reacting the residual mass obtained in step (b) in an ether solvent with diethyl(3-pyridyl)borane in the presence of bis(triphenylphosphine)palladium(II) chloride;
d) adding a base and water to the reaction mass;
e) concentrating the reaction mass to obtain a residual mass;
f) adding a base and an alcoholic solvent to the residual mass obtained in step (e);
g) adding water to the reaction mass;
h) pH of the reaction mass was adjusted with hydrochloric acid;
i) adding chlorinated solvent to the reaction mass;
j) removing the solvent from the reaction mass to obtain a residual solid;
k) slurring the residual solid obtained in step (j) with a hydrocarbon solvent; and
l) isolating the abiraterone.

The chlorinated solvent used in step (a) and (i) may preferably be a solvent or a mixture of solvents selected from methylene chloride, chloroform, carbontetrachloride and ethylene dichloride, and more preferable chlorinated solvent is methylene chloride.

Preferably the reaction mass is concentrated in step (b) and (e) by distilling off the solvent. The distilling off the solvent may be carried out at atmospheric pressure or at reduced pressure. The distillation may preferably be carried out until the solvent is almost completely distilled off.

The ether solvent used in step (c) may preferably be a solvent or a mixture of solvents selected from tetrahydrofuran, methyl tetrahydrofuran, methyl tert-butyl ether, ethyl tert-butyl ether, 1,4-dioxane, diisopropyl ether, diethyl ether and tetrahydropyran. More preferably the ether solvent is tetrahydrofuran.

Preferably the base used in step (d) and (f) may be organic base or inorganic base and more preferable base is inorganic base selected from alkali metal hydroxides, alkali metal carbonates or alkali metal bicarbonates. Still more preferably the base is sodium bicarbonate or sodium hydroxide.

The alcoholic solvent used in step (f) may preferably be a solvent or a mixture of solvents selected from methanol, ethanol, isopropanol and n-butanol. More preferably the alcoholic solvent is methanol.

Removal of the solvent may be carried out in step (j) at atmospheric pressure or at reduced pressure. Removal of the solvent may preferably be carried out until the solvent is almost completely distilled off.

The hydrocarbon solvent used in step (k) may preferably be a solvent or a mixture of solvents selected from hexane, cyclohexane, n-hexane, heptane, benzene, toluene and xylene. More preferably the hydrocarbon solvent is toluene.

Isolation of abiraterone in step (1) may preferably be performed by conventional techniques such as centrifugation and filtration.

According to another aspect of the present invention, there is provided a novel process for the preparation of abiraterone acetate, which comprises the steps (a) to (1) as defined above and which further comprises:
a) reacting abiraterone in a chlorinated solvent with acetic anhydride in the presence of pyridine and 4-dimethylaminopyridine;
b) adding sodium bicarbonate solution to the reaction mass;
c) concentrating the reaction mass to obtain a residual solid;
d) dissolving the residual solid in a nitrile solvent, an alcoholic solvent or mixture thereof;
e) heating the solution; and
f) isolating the abiraterone acetate.

The chlorinated solvent used in step (a) may preferably be a solvent or a mixture of solvents selected from methylene chloride, chloroform, carbontetrachloride and ethylene dichloride, and more preferably the chlorinated solvent is methylene chloride.

Preferably the reaction mass is concentrated in step (c) by distilling off the solvent. The distilling off the solvent may be carried out at atmospheric pressure or at reduced pressure. The distillation may preferably be carried out until the solvent is almost completely distilled off.

The nitrile solvent used in step (d) may preferably be a solvent or a mixture of solvents selected from acetonitrile, propionitrile, butyronitrile and benzonitrile, and more preferably the nitrile solvent is acetonitrile.

The alcoholic solvent used in step (d) may preferably be a solvent or a mixture of solvents selected from methanol, ethanol, isopropanol and n-butanol. More preferably the alcoholic solvent is methanol.

Abiraterone acetate may be isolated in step (f) by methods known such as filtration or centrifugation.

An embodiment of the present invention, there is provided crystalline particles of abiraterone acetate having mean particle size (D₅₀) ranging from about 1 µm to 10 µm and 90 volume-% of the particles (D₉₀) ranging from about 5 µm to 30 µm.

The term "µm" refers to "micrometer" which is 1x10⁻⁶ meter.

The term "crystalline particles" means any combination of single crystals, aggregates and agglomerates.

The term "Particle Size Distribution (P.S.D.)" means the cumulative volume size distribution of equivalent spherical diameters as determined by laser diffraction at 1 bar dispersive pressure in Sympatec Helos equipment. "Mean particle size distribution, i.e., D₅₀" correspondingly, means the median of said particle size distribution.

The invention will now be further described by the following example, which is illustrative rather than limiting.

### Examples

### Example 1:

### Preparation of abiraterone

Dehydroepiandrosterone-3-acetate (200 gm) was dissolved in methylene chloride (2000 ml) at 0 to 5°C and then added trifluoromethanesulfonic anhydride (256 gm). To the contents were added a solution of tetramethylethylenediamine (57 gm) in methylene chloride (500 ml) slowly for 30 minutes at 0 to 5°C and stirred for 3 hours. The temperature of the reaction mass was raised to room temperature and then added water (3000 ml). The layers were separated and the aqueous layer was extracted with methylene chloride. Combined organic layers were dried with sodium sulfate and then concentrated to obtain a residual mass. The residual mass obtained was dissolved in tetrahydrofuran (2000 ml) and then added diethyl(3-pyridyl)borane (76 gm) at room temperature. The reaction mass was stirred for 30 minutes and then added bis(triphenylphosphine)palladium(II) chloride (2.5 gm), and stirred for 30 minutes. A solution of sodium carbonate (205 gm) in water (1000 ml) added to the reaction mass under nitrogen atmosphere. The reaction mass was stirred for 30 minutes at room temperature and then heated to 80°C. The reaction mass was maintained for 5 hours 30 minutes at 80°C and then cooled to room temperature. Water (3000 ml) and ethyl acetate (2000 ml) was added to the reaction mass and then the layers were separated. The aqueous layer was extracted with ethyl acetate. Combined organic layers were dried with sodium sulfate and then concentrate to obtain a residual mass. The residual mass obtained was dissolved in methanol (2400 ml) and then added sodium hydroxide solution (10%; 500 ml). The contents were heated to 80°C and maintained for 2 hours. The reaction mass was then cooled to room temperature and then added water (1200 ml). The reaction mass was then cooled to 15 to 20°C and pH was adjusted to 5.5 with hydrochloric acid (2N). To the reaction mass was added methylene chloride (3000 ml) and the separated aqueous layer was extracted with methylene chloride. Combined organic layers were dried with sodium sulfate and then concentrate to obtain a residual solid. To the residual solid was added toluene (1600 ml) and then heated to 90°C for 15 minutes. The solution was then cooled to room temperature and stirred for 3 hours. The contents were further cooled to 0 to 5°C, stirred for 30 minutes and filtered. The solid obtained was then dried to obtain 125 gm of abiraterone.

### Example 2:

### Preparation of abiraterone

Dehydroepiandrosterone-3-acetate (100 gm) was dissolved in methylene chloride (1000 ml) at 0 to 5°C and then added trifluoromethanesulfonic anhydride (128 gm). To the contents were added a solution of n-methylpyrrolidone (21 gm) in methylene chloride (250 ml) slowly for 30 minutes at 0 to 5°C and stirred for 3 hours. The temperature of the reaction mass was raised to room temperature and then added water (1500 ml). The layers were separated and the aqueous layer was extracted with methylene chloride. Combined organic layers were dried with sodium sulfate and then concentrated to obtain a residual mass. The residual mass obtained was dissolved in tetrahydrofuran (1000 ml) and then added diethyl(3-pyridyl)borane (38 gm) at room temperature. The reaction mass was stirred for 30 minutes and then added bis(triphenylphosphine)palladium(II) chloride (1.2 gm), and stirred for 30 minutes. A solution of sodium carbonate (102 gm) in water (500 ml) added to the reaction mass under nitrogen atmosphere. The reaction mass was stirred for 30 minutes at room temperature and then heated to 80°C. The reaction mass was maintained for 5 hours 30 minutes at 80°C and then cooled to room temperature. Water (1500 ml) and ethyl acetate (1000 ml) was added to the reaction mass and then the layers were separated. The aqueous layer was extracted with ethyl acetate. Combined organic layers were dried with sodium sulfate and then concentrate to obtain a residual mass. The residual mass obtained was dissolved in methanol (1200 ml) and then added sodium hydroxide solution (10%; 250 ml). The contents were heated to 80°C and maintained for 2 hours. The reaction mass was then cooled to room temperature and then added water (600 ml). The reaction mass was then cooled to 15 to 20°C and pH was adjusted to 5.5 with hydrochloric acid (2N). To the reaction mass was added methylene chloride (1500 ml) and the separated aqueous layer was extracted with methylene chloride. Combined organic layers were dried with sodium sulfate and then concentrate to obtain a residual solid. To the residual solid was added toluene (800 ml) and then heated to 90°C for 15 minutes. The solution was then cooled to room temperature and stirred for 3 hours. The contents were further cooled to 0 to 5°C, stirred for 30 minutes and filtered. The solid obtained was then dried to obtain 58 gm of abiraterone.

### Example 3:

### Preparation of abiraterone

Example 1 was repeated using N-methylpiperidine base instead of tetramethylethylenediamine base to obtain abiraterone.

### Example 4:

### Preparation of abiraterone acetate

Abiraterone (90 gm) was dissolved in methylene chloride (1080 ml) and then added pyridine (204 gm) and acetic anhydride (132 gm) at room temperature. To the reaction mixture was added 4-dimethylaminopyridine (4.5 gm) and stirred for 4 hours at room temperature. Sodium bicarbonate (7%, 900 ml) solution was added to the reaction mass and then the layers were separated. The organic layer was dried with sodium sulfate and then concentrated to obtain a residual solid. To the residual solid was added acetonitrile (360 ml) and methanol (90 ml) at room temperature and then heated to 65 to 70°C for 15 minutes. The solution was then cooled to room temperature and stirred for 14 hours. The separated solid was filtered and then dried to obtain 70 gm of abiraterone acetate. [Mean particle size (D₅₀): 15.61 µm and 90 volume-% of the particles (D₉₀): 38.75 µm]

### Example 5:

### Preparation of abiraterone acetate

Abiraterone acetate as obtained in example 4 was micronized to obtain abiraterone acetate having a mean particle size (D₅₀): 2.99 µm and and 90 volume-% of the particles (D₉₀): 6.29 µm.

## Claims

1. A process for the preparation of abiraterone, which comprises:
a) reacting dehydroepiandrosterone-3-acetate in a chlorinated solvent with trifluoromethanesulfonic anhydride in the presence of a base selected from the group consisting of n-methylpyrrolidone, N-methylpiperidine or tetramethylethylenediamine in a chlorinated solvent;
b) concentrating the reaction mass to obtain a residual mass;
c) reacting the residual mass obtained in step (b) in an ether solvent with diethyl(3-pyridyl)borane in the presence of bis(triphenylphosphine)palladium(II) chloride;
d) adding a base and water to the reaction mass;
e) concentrating the reaction mass to obtain a residual mass;
f) adding a base and an alcoholic solvent to the residual mass obtained in step (e);
g) adding water to the reaction mass;
h) pH of the reaction mass was adjusted with hydrochloric acid;
i) adding chlorinated solvent to the reaction mass;
j) removing the solvent from the reaction mass to obtain a residual solid;
k) slurring the residual solid obtained in step (j) with a hydrocarbon solvent; and
l) isolating the abiraterone.

2. The process as claimed in claim 1, wherein the chlorinated solvent used in step (a) and (i) is a solvent or a mixture of solvents selected from methylene chloride, chloroform, carbontetrachloride and ethylene dichloride.

3. The process as claimed in claim 1, wherein the ether solvent used in step (c) is a solvent or a mixture of solvents selected from tetrahydrofuran, methyl tetrahydrofuran, methyl tert-butyl ether, ethyl tert-butyl ether, 1,4-dioxane, diisopropyl ether, diethyl ether and tetrahydropyran.

4. The process as claimed in claim 1, wherein the base used in step (d) and (f) is organic base or inorganic base.

5. The process as claimed in claim 4, wherein the base is inorganic base selected from alkali metal hydroxides, alkali metal carbonates or alkali metal bicarbonates.

6. The process as claimed in claim 5, wherein the base is sodium bicarbonate or sodium hydroxide.

7. The process as claimed in claim 1, wherein the alcoholic solvent used in step (f) is a solvent or a mixture of solvents selected from methanol, ethanol, isopropanol and n-butanol.

8. The process as claimed in claim 1, wherein the hydrocarbon solvent used in step (k) is a solvent or a mixture of solvents selected from hexane, cyclohexane, n-hexane, heptane, benzene, toluene and xylene.

9. A process for the preparation of abiraterone acetate, which comprises carrying out the process as defined in claim 1, and which further comprises:
a) reacting abiraterone in a chlorinated solvent with acetic anhydride in the presence of pyridine and 4-dimethylaminopyridine;
b) adding sodium bicarbonate solution to the reaction mass;
c) concentrating the reaction mass to obtain a residual solid;
d) dissolving the residual solid in a nitrile solvent, an alcoholic solvent or mixture thereof;
e) heating the solution; and
f) isolating the abiraterone acetate.

10. The process as claimed in claim 9, wherein the chlorinated solvent used in step (a) is a solvent or a mixture of solvents selected from methylene chloride, chloroform, carbontetrachloride and ethylene dichloride.

11. The process as claimed in claim 9, wherein the nitrile solvent used in step (d) is a solvent or a mixture of solvents selected from acetonitrile, propionitrile, butyronitrile and benzonitrile.

12. The process as claimed in claim 9, wherein the alcoholic solvent used in step (d) is a solvent or a mixture of solvents selected from methanol, ethanol, isopropanol and n-butanol.

## Patentansprüche

1. Verfahren zur Herstellung von Abirateron, umfassend:
a) das Umsetzen von Dehydroepiandrosteron-3-acetat in einem chlorierten Lösungsmittel mit Trifluormethansulfonsäureanhydrid in Gegenwart einer Base, ausgewählt aus der Gruppe, bestehend aus n-Methylpyrrolidon, N-Methylpiperidin oder Tetramethylethylendiamin in einem chlorierten Lösungsmittel;
b) das Konzentrieren der Reaktionsmasse unter Erhalt einer Restmasse;
c) das Umsetzen der in Schritt (b) erhaltenen Restmasse in einem Ether-Lösungsmittel mit Diethyl(3-pyridyl)boran in Gegenwart von Bis(triphenylphosphin)palladium(II)chlorid;
d) das Zugeben einer Base und von Wasser zu der Reaktionsmasse;
e) das Konzentrieren der Reaktionsmasse unter Erhalt einer Restmasse;
f) das Zugeben einer Base und eines alkoholischen Lösungsmittels zu der in Schritt (e) erhaltenen Restmasse;
g) das Zugeben von Wasser zu der Reaktionsmasse;
h) das Einstellen des pH der Reaktionsmasse mit Salzsäure;
i) das Zugeben eines chlorierten Lösungsmittels zu der Reaktionsmasse;
j) das Entfernen des Lösungsmittels aus der Reaktionsmasse unter Erhalt eines festen Restes;
k) das Aufschlämmen des in Schritt (j) erhaltenen festen Restes mit einem Kohlenwasserstoff-Lösungsmittel und
l) das Isolieren des Abiraterons.

2. Verfahren nach Anspruch 1, wobei das in Schritt (a) und (i) verwendete chlorierte Lösungsmittel ein Lösungsmittel oder ein Gemisch von Lösungsmitteln, ausgewählt aus Methylenchlorid, Chloroform, Kohlenstofftetrachlorid und Ethylendichlorid, ist.

3. Verfahren nach Anspruch 1, wobei das in Schritt (c) verwendete Ether-Lösungsmittel ein Lösungsmittel oder ein Gemisch von Lösungsmitteln, ausgewählt aus Tetrahydrofuran, Methyltetrahydrofuran, Methyl-tert-butylether, Ethyl-tert-butylether, 1,4-Dioxan, Diisopropylether, Diethylether und Tetrahydropyran, ist.

4. Verfahren nach Anspruch 1, wobei die in Schritt (d) und (f) verwendete Base eine organische Base oder eine anorganische Base ist.

5. Verfahren nach Anspruch 4, wobei die Base eine anorganische Base, ausgewählt aus Alkalimetallhydroxiden, Alkalimetallcarbonaten oder Alkalimetallbicarbonaten, ist.

6. Verfahren nach Anspruch 5, wobei die Base Natriumbicarbonat oder Natriumhydroxid ist.

7. Verfahren nach Anspruch 1, wobei das in Schritt (f) verwendete alkoholische Lösungsmittel ein Lösungsmittel oder ein Gemisch von Lösungsmitteln, ausgewählt aus Methanol, Ethanol, Isopropanol und n-Butanol, ist.

8. Verfahren nach Anspruch 1, wobei das in Schritt (k) verwendete KohlenwasserstoffLösungsmittel ein Lösungsmittel oder ein Gemisch von Lösungsmitteln, ausgewählt aus Hexan, Cyclohexan, n-Hexan, Heptan, Benzol, Toluol und Xylol, ist.

9. Verfahren zur Herstellung von Abirateronacetat, welches das Durchführen des wie in Anspruch 1 definierten Prozesses umfasst und welches ferner:
a) das Umsetzen von Abirateron in einem chlorierten Lösungsmittel mit Essigsäureanhydrid in Gegenwart von Pyridin und 4-Dimethylaminopyridin;
b) das Zugeben von Natriumbicarbonatlösung zu der Reaktionsmasse;
c) das Konzentrieren der Reaktionsmasse unter Erhalt eines festen Restes;
d) das Lösen des festen Restes in einem Nitril-Lösungsmittel, einem alkoholischen Lösungsmittel oder einem Gemisch davon;
e) das Erhitzen der Lösung und
f) das Isolieren des Abirateronacetats
umfasst.

10. Verfahren nach Anspruch 9, wobei das in Schritt (a) verwendete chlorierte Lösungsmittel ein Lösungsmittel oder ein Gemisch von Lösungsmitteln, ausgewählt aus Methylenchlorid, Chloroform, Kohlenstofftetrachlorid und Ethylendichlorid, ist.

11. Verfahren nach Anspruch 9, wobei das in Schritt (d) verwendete Nitril-Lösungsmittel ein Lösungsmittel oder ein Gemisch von Lösungsmitteln, ausgewählt aus Acetonitril, Propionitril, Butyronitril und Benzonitril, ist.

12. Verfahren nach Anspruch 9, wobei das in Schritt (d) verwendete alkoholische Lösungsmittel ein Lösungsmittel oder ein Gemisch von Lösungsmitteln, ausgewählt aus Methanol, Ethanol, Isopropanol und n-Butanol, ist.

## Revendications

1. Procédé de préparation de l'abiratérone, qui comprend :
a) la réaction du 3-acétate de déshydroépiandrostérone dans un solvant chloré avec l'anhydride trifluoro-méthanesulfonique en présence d'une base choisie dans le groupe constitué par la n-méthylpyrrolidone, la N-méthylpipéridine ou la tétraméthyléthylènediamine dans un solvant chloré ;
b) la concentration de la masse réactionnelle pour obtenir une masse résiduelle ;
c) la réaction de la masse résiduelle obtenue dans l'étape (b) dans un solvant de type éther avec le (3-pyridyl)borane de diéthyle en présence de chlorure de bis(triphénylphosphine)palladium (II) ;
d) l'ajout d'une base et d'eau à la masse réactionnelle ;
e) la concentration de la masse réactionnelle pour obtenir une masse résiduelle ;
f) l'ajout d'une base et d'un solvant alcoolique à la masse résiduelle obtenue dans l'étape (e) ;
g) l'ajout d'eau à la masse réactionnelle ;
h) le pH de la masse réactionnelle a été ajusté avec de l'acide chlorhydrique ;
i) l'ajout d'un solvant chloré à la masse réactionnelle ;
j) l'élimination du solvant à partir de la masse réactionnelle pour obtenir un solide résiduel ;
k) la mise en suspension du solide résiduel obtenu dans l'étape (j) dans un solvant hydrocarboné ; et
l) l'isolement l'abiratérone.

2. Procédé selon la revendication 1, dans lequel le solvant chloré utilisé dans les étapes (a) et (i) est un solvant ou un mélange de solvants choisi parmi le chlorure de méthylène, le chloroforme, le tétrachlorure de carbone et le dichlorure d'éthylène.

3. Procédé selon la revendication 1, dans lequel le solvant de type éther utilisé dans l'étape (c) est un solvant ou un mélange de solvants choisi parmi le tétrahydrofurane, le méthyl tétrahydrofurane, le méthyl tert-butyl éther, l'éthyl tert-butyl éther, le 1,4-dioxane, le diisopropyl éther, le diéthyl éther et le tétrahydropyrane.

4. Procédé selon la revendication 1, dans lequel la base utilisée dans les étapes (d) et (f) est une base organique ou une base inorganique.

5. Procédé selon la revendication 4, dans lequel la base est une base inorganique choisie parmi les hydroxydes de métaux alcalins, les carbonates de métaux alcalins ou les bicarbonates de métaux alcalins.

6. Procédé selon la revendication 5, dans lequel la base est le bicarbonate de sodium ou l'hydroxyde de sodium.

7. Procédé selon la revendication 1, dans lequel le solvant alcoolique utilisé dans l'étape (f) est un solvant ou un mélange de solvants choisi parmi le méthanol, l'éthanol, l'isopropanol et le n-butanol.

8. Procédé selon la revendication 1, dans lequel le solvant hydrocarboné utilisé dans l'étape (k) est un solvant ou un mélange de solvants choisi parmi l'hexane, le cyclohexane, le n-hexane, l'heptane, le benzène, le toluène et le xylène.

9. Procédé de préparation de l'acétate d'abiratérone, qui comprend la mise en oeuvre du procédé tel que défini dans la revendication 1, et qui comprend en outre :
a) la réaction de l'abiratérone dans un solvant chloré avec de l'anhydride acétique en présence de pyridine et de 4-diméthylaminopyridine ;
b) l'ajout d'une solution de bicarbonate de sodium à la masse réactionnelle ;
c) la concentration de la masse réactionnelle pour obtenir un solide résiduel ;
d) la dissolution du solide résiduel dans un solvant de type nitrile, un solvant alcoolique ou un mélange de ceux-ci ;
e) le chauffage de la solution ; et
f) l'isolement de l'acétate d'abiratérone.

10. Procédé selon la revendication 9, dans lequel le solvant chloré utilisé dans l'étape (a) est un solvant ou un mélange de solvant choisi parmi le chlorure de méthylène, le chloroforme, le tétrachlorure de carbone et le dichlorure d'éthylène.

11. Procédé selon la revendication 9, dans lequel le solvant de type nitrile utilisé dans l'étape (d) est un solvant ou un mélange de solvant choisi parmi l'acétonitrile, le propionitrile, le butyronitrile et le benzonitrile.

12. Procédé selon la revendication 9, dans lequel le solvant alcoolique utilisé dans l'étape (d) est un solvant ou un mélange de solvant choisi parmi le méthanol, l'éthanol, l'isopropanol et le n-butanol.
